# EUROPEAN PATENT SPECIFICATION

(11) **EP 1 833 425 B1**
(45) Date of publication and mention of the grant of the patent: **23.02.2011**
(21) Application number: 05852610.4
(22) Date of filing: 01.12.2005
(51) Int. Cl.: A61F 2/24, A61F 2/84

(54) **SENSING DELIVERY SYSTEM FOR INTRALUMINAL MEDICAL DEVICES**
MESSFÄHIGES ABGABESYSTEM FÜR INTRALUMINALE MEDIZINPRODUKTE
SYSTEME A DETECTION DE POSE POUR DISPOSITIFS MEDICAUX INTRALUMINAUX

(30) Priority: 01.12.2004 US 523000 P
(43) Date of publication of application: 19.09.2007
(73) Proprietor: Cook Incorporated, Bloomington, IN 47404 (US)
(72) Inventor: CASE, Brian, C., Lake Villa, Illinois 60046 (US)
(74) Representative: Garratt, Peter Douglas
(86) International application number: PCT/US2005/043424
(87) International publication number: WO 2006/073628

(56) References cited:
- EP-A- 1 472 996
- WO-A-02/07601
- WO-A-98/19732
- WO-A-99/33414
- US-A- 5 053 008
- US-A- 5 928 248
- US-A1- 2003 125 790
- US-A1- 2004 073 238
- US-A1- 2004 102 806
- US-A1- 2005 149 159
- US-A1- 2005 192 496
- US-B1- 6 179 858
- US-B1- 6 632 196

## Description

### CROSS-REFERENCE TO RELATED APPLICATION

This application claims priority to United States Provisional Application Serial No. 60/523,000, filed on December 1, 2004.

### FIELD

The present application for patent relates to medical devices. Exemplary embodiments described herein relate to delivery systems for implantation of intraluminal medical devices in a body vessel and methods of implanting intraluminal medical devices.

### BACKGROUND

Minimally invasive techniques and instruments for placement of intraluminal medical devices have been developed over recent years and are frequently used to deliver an intraluminal medical device to a desired point of treatment and deploy the intraluminal medical device at the point of treatment. In these techniques, a delivery system is used to carry the intraluminal medical device through a body vessel and to the point of treatment. Once the point of treatment is reached, the intraluminal medical device is deployed from the delivery system. The delivery system is subsequently withdrawn from the point of treatment and, ultimately, the body vessel. A wide variety of treatment devices that utilize minimally invasive technology have been developed and include stents, stent grafts, occlusion devices, infusion catheters, prosthetic valves, and the like.

For some intraluminal medical devices, it may be desirable to observe the point of treatment prior to delivery of the intraluminal medical device. Such an observation is shown and described in U.S. Pat. Appl. Pub. No. 2003/0199768 to Cespedes et al. for METHODS AND APPARATUS FOR THE IDENTIFICATION AND STBILIZATION OF VULNERABLE PLAQUE.

This pre-deployment observation can ensure that the point of treatment is in suitable condition to receive the intraluminal medical device.

For other intraluminal medical devices, it may be desirable to assess one or more parameters of the body vessel and/or body fluid within the body vessel prior to deployment of the intraluminal medical device at a point of treatment. For example, it may be desirable to measure vessel diameter and/or fluid pressure priorto deployment. Furthermore, it may be desirable to assess one or more vessel and/or fluid parameters after deployment of an intraluminal medical device at a point of treatment. Such an assessment may aid in verifying function and/or placement of the intraluminal medical device.

Accordingly, there is a need for a delivery system which facilitates assessment of one or more vessel and/or fluid parameters prior to, during, and/or following deployment of an intraluminal medical device at a point of treatment within a body vessel.

A similar device is described in US 6 632 116 B1.

### SUMMARY OF EXEMPLARY EMBODIMENTS

Delivery systems useful in the implantation of intraluminal medical devices at a point of treatment in a body vessel are provided. The invention is defined in claim 1. The delivery systems include a sensing apparatus that allows a user to gather information relating to vessel and/or fluid parameters. The information can be used for a variety of purposes, such as confirmation of vessel sizing and verification of function of an implanted intraluminal medical device. The information can also be used to determine if additional steps are necessary for the implantation procedure.

A delivery system according to an exemplary embodiment of the invention comprises a tubular member, a dilator disposed in the tubular member and an intraluminal medical device disposed in a device chamber formed between the dilator and the tubular member. A sensing apparatus is disposed in the distal end of the dilator and is adapted to determine at least one of a vessel parameter and a fluid parameter prior to deployment, during deployment, and/or after deployment of the intraluminal medical device.

Methods of implanting an intraluminal medical device are also described. One exemplary method comprises an initial step of providing a delivery system that includes an intraluminal medical device and a sensing apparatus disposed in the distal end of a dilator. The sensing apparatus is adapted to determine at least one of a vessel parameter and a fluid parameter prior to deployment, during deployment, and/or after deployment of the intraluminal medical device. Another step comprises inserting a distal end of the delivery system into a body vessel of a patient. Another step comprises determining at least one of a vessel parameter and a fluid parameter using the sensing apparatus. Another step comprises deploying the intraluminal medical device. Another step comprises removing the delivery system from the body vessel of the patient. The step of determining at least one of a vessel parameter and a fluid parameter using the sensing apparatus can be conducted prior to, during, and/or after the step of deploying the intraluminal medical device.

In exemplary embodiments of the delivery system the intraluminal medical device comprises a valve medical device, such as a venous valve device and a heart valve device.

Additional understanding of the invention can be obtained with review of the description of exemplary embodiments of the invention, appearing below, and the appended drawings that illustrate exemplary embodiments.

### BRIEF DESCRIPTION OF THE DRAWINGS

Figure 1 is a perspective view of a delivery system according to an exemplary embodiment.

Figure 2 is a partial sectional view of the distal end of the delivery system illustrated in Figure 1.

Figure 3 is a partial sectional view of a body vessel containing the delivery system of Figure 1 prior to deployment of an intraluminal medical device.

Figure 4 is a partial sectional view of a body vessel containing the delivery system of Figure 1 during a first stage of deployment of an intraluminal medical device.

Figure 5 is a partial sectional view of a body vessel containing the delivery system of Figure 1 during a second stage of deployment of an intraluminal medical device.

Figure 6 is a partial sectional view of a body vessel containing the delivery system of Figure 1 during a third stage of deployment of an intraluminal medical device.

Figure 7 is a block diagram illustrating a method of implanting an intraluminal medical device according to an exemplary embodiment.

Figure 8 is a partial sectional view of the distal end of a delivery system according to another exemplary embodiment.

### DETAILED DESCRIPTION OF EXEMPLARY EMBODIMENTS

The following detailed description and appended drawings describe and illustrate various exemplary embodiments. The description and drawings serve to enable one skilled in the art to make and use the invention, and are not intended to limit the scope of the invention in any manner.

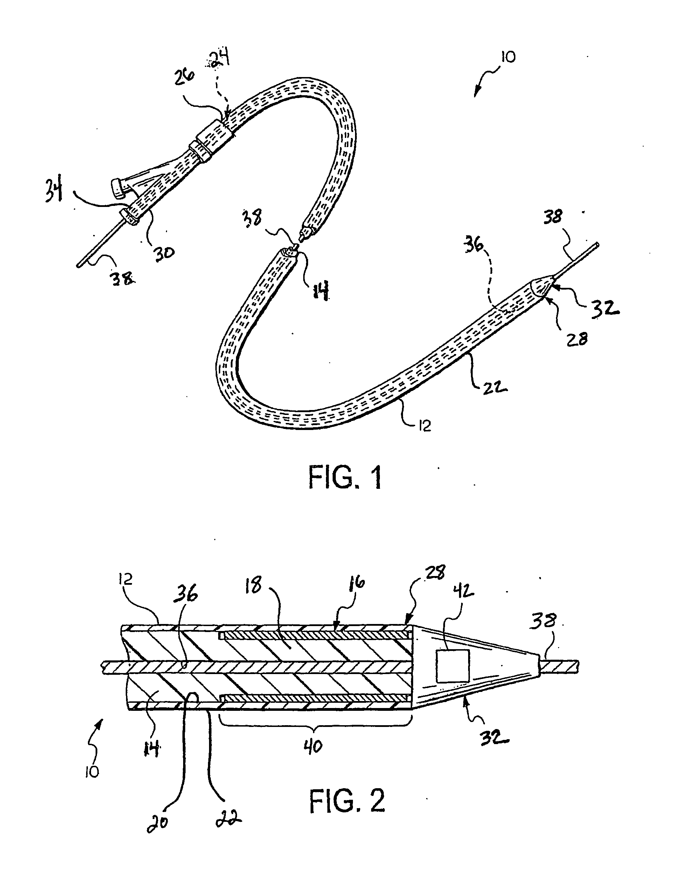

Figures 1 through 6 illustrate a delivery system 10 according to a first exemplary embodiment. The delivery system 10 includes a tubular member 12 and a dilator 14 disposed within the tubular member 12. The tubular member 12, in effect, serves as a sheath disposed over the dilator 14. An intraluminal medical device 16 is disposed on a distal end 18 of the dilator 14 and can be deployed at a point of treatment in a body vessel following retraction of the tubular member 12 to a point proximal of the intraluminal medical device 16.

It is noted that while the intraluminal medical device 16 is illustrated as a self-expandable device, it is understood that balloon expandable, and indeed any type of intraluminal medical device, can be used with delivery systems according to the invention. For the illustrated embodiment, the intraluminal medical device 16 is deployed by self-expansion following retraction of the tubular member 12 to a point proximal of the intraluminal medical device 16. If a balloon-expandable intraluminal medical device is utilized, a force is applied, such as by inflation of an underlying balloon, to affect expansion of the intraluminal medical device following retraction of the tubular member 12.

The tubular member has inner 20 and outer 22 surfaces and defines a passageway 24 extending from a proximal end 26 to a distal end 28. The passageway 24 provides a space within which other components of the delivery system 10 can be disposed. The proximal end 26 can include any desirable connectors and/or adaptors, such as a threaded fitting, Touhy-Borst adapter 30, and other suitable connectors and adaptors. Also, a handle or handle system configured to allow sliding of the dilator 14 relative to the tubular member 12, or vice versa, can be attached to the proximal end 26 of the tubular member 12. These elements, however, are not required, and the tubular member 12 can indeed comprise a simple tubular body.

The tubular member can be any suitable tubular member and need only provide a passageway into which a dilator, such as dilator 14, can be disposed. Any suitable material can be used to form the tubular member 12. Examples of suitable materials include polypropylene, polyurethane, nylon, and other polymeric materials. Also, tubular members comprising multiple materials can be used. For example, a tubular member that includes a reinforcing coil or strand disposed in or on the material of the tubular member can be used.

The dilator 14 is disposed within the passageway 24 of the tubular member 12. As used herein, the term "dilator" refers to an elongate member capable of being disposed within a lumen of a sheath, such as the tubular member 12. The dilator 14 has a tapered distal tip 32 and a proximal end 34. A lumen 36 is formed by the dilator 14 and extends along the entire length of the dilator 14. The lumen 36 is adapted to receive a guiding member, such as wireguide 38 or other suitable guiding member. The lumen 36 may aid in guiding the delivery system 10 over the wireguide 38 to a desired point of treatment. As used herein, the term "wireguide" refers to an elongate member used in a minimally invasive procedure to define a path along which other devices can be advanced. The term is considered equivalent in meaning to the term "guidewire" as also used in the art. The term does not require any particular material in the composition of the guiding member.

While the illustrated embodiment is adapted for over-the-wire applications, it is expressly understood that modification of the delivery system for use in rapid exchange applications, such as by modifying the length of the wireguide lumen 36 to a length that extends along only a portion of the length of the dilator 14, is within the scope of the invention.

Figure 2 illustrates the distal end of the delivery system 10. Intraluminal medical device 16 is disposed in a device chamber 40 formed in the distal end 18 of the dilator 14. As best illustrated in Figure 2, the device chamber 40 is advantageously positioned proximal to the tapered distal tip 32 of the dilator 14. A portion of the tubular member 12 is disposed about the intraluminal medical device 16 and protects the intraluminal medical device 16 from the external environment. For self-expandable intraluminal medical devices, the portion of the tubular member 12 that is disposed about the intraluminal medical device 16 provides the constraining force necessary to maintain the intraluminal medical device 16 in an unexpanded configuration until deployment is desired.

The intraluminal medical device 16 can be any suitable intraluminal medical device and the type of intraluminal medical device used in a delivery system according to a particular embodiment of the invention will depend at least upon the clinical situation in which the delivery system is being used. Exemplary types of intraluminal medical devices suitable for use in delivery systems according to the invention include stents, prosthetic valves, filters, occluders, distal protection devices, stent grafts, and the like. Examples of suitable intraluminal medical devices for use in and with devices according to the invention include those described in United States Patents 6,464,720 to Boatman et al. for a RADIALLY EXPANDABLE STENT; 6,231,598 to Berry et al. for a RADIALLY EXPANDABLE STENT; 6,299,635 to Frantzen for a RADIALLY EXPANDABLE NON-AXIALLY CONTRACTING SURGICAL STENT; and 5,580,568 to Gianturco for a PERCUTANEOUS ENDOVASCULAR STENT AND METHOD FOR INSERTION THEREOF.

As described more fully below, delivery systems according to the invention are particularly well-suited for use with intraluminal medical devices for which verification of placement, position, and/or function following deployment may be desirable. According to the invention medical devices are valve medical devices. Following implantation of a valve device, it may be desirable to verify valve placement, position, and/or function. The delivery systems according to the invention can be used with any suitable valve device, including venous valve devices and heart valve devices. Examples of suitable venous valve devices are described in United States Patents 6,508,833 to Pavcnik et al. for a MULITPLE-SIDED INTRALUMINAL MEDICAL DEVICE and published application for United States patent 20010039450 to Pavcnik et al. for an IMPLANTABLE MEDICAL DEVICE. Examples of suitable heart valve devices are described in United States Patents 6,767,362 to Schreck for MINIMALLY INVASIVE HEART VALVES AND METHODS OF USE and 6,733,525 to Yang et al. for ROLLED MINIMALLY INVASIVE HEART VALVES AND METHODS OF USE.

A sensing apparatus 42 is disposed in the distal tip 32 of the dilator 14. The sensing apparatus is a means for determining a vessel parameter and/or a means for determining a fluid parameter. Any suitable means for determining can be used, and exemplary means for determining include imaging apparatuses, such as an intravascular ultrasound (IVUS) system, a fiber optic visualization system, an infrared imaging system, and an ultrasound transducer, including linear-array, phased-array, rotational, forward-looking, and radial-looking ultrasound transducers. Other exemplary imaging apparatuses include a magnetic resonance imaging apparatus, an angiography apparatus, an optical coherence tomography apparatus, and combinations of two or more imaging apparatuses. Other exemplary means for determining include fluid pressure sensors, biochemical sensors, such as pH sensors able to determine a pH measurement of a fluid in a body vessel, and temperature sensors.

Exemplary vessel parameters for determination by the means for determining include vessel dimensions, including the inner diameter of a body vessel, and visual appearance of the vessel or portions of the vessel. Exemplary fluid parameters for determination by the means for determining include fluid pressure, the presence and/or lack of fluid flow, the velocity of fluid flow, fluid temperature, and fluid pH.

United States Patent Application Publication Numbers 2003/0199768 to Cespedes et al. for METHODS AND APPARATUS FOR THE IDENTIFICATION AND STABILIZATION OF VULNERABLE PLAQUE; 2003/0199747 to Michlitsch et al. for METHODS AND APPARATUS FOR THE IDENTIFICATION AND STABILIZATION OF VULNERABLE PLAQUE; 2003/0199767 to Cespedes et al. for METHODS AND APPARATUS FOR THE IDENTIFICATION AND STABILIZATION OF VULNERABLE PLAQUE; and 2003/0236443 to Cespedes et al. for METHODS AND APPARATUS FOR THE IDENTIFICATION AND STABILIZATION OF VULNERABLE PLAQUE describe several suitable means for determining that can be used as a means for determining a vessel parameter and/or a means for determining a fluid parameter in a delivery system according to the present invention.

While the illustrated embodiment includes the sensing apparatus 42 in the distal tip 32 of the dilator 14, it is understood that the sensing apparatus 42 can be disposed at any suitable location in or on the dilator 14. Placement in the distal tip 32 is considered advantageous at least because of the proximity of the distal tip 32 to the intraluminal medical device 16, both prior to and during deployment, as will be described more fully below. According to the invention the position for the sensing apparatus 42 is a position in or on the dilator 14 adjacent the device chamber 40 and a position in or on the dilator 14 spaced a desired distance from the distal tip 32 of the dilator 14.

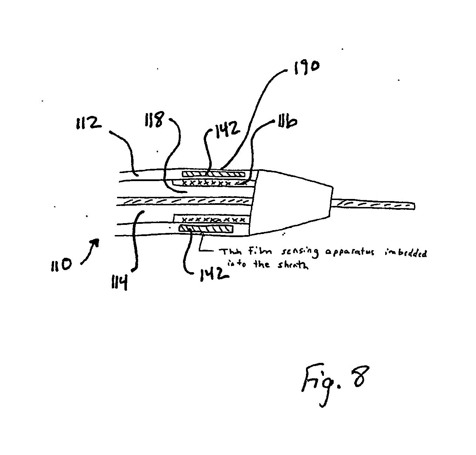

Figure 8 illustrates a delivery system 110 according to another exemplary embodiment. The delivery system 110 of this embodiment is identical to the embodiment illustrated in Figures 1 through 6, except as described below. Accordingly, the delivery system 110 includes a tubular member 112 and a dilator 114 disposed within the tubular member 112. An intraluminal medical device 116 is disposed on a distal end 118 of the dilator 114. In this embodiment, a sensing apparatus 142 is associated with the distal end 190 of the tubular member 112. The sensing apparatus 142 can be disposed on any surface of the tubular member 112 or can be embedded within the tubular member 112, as illustrated in the Figure. Also, the sensing apparatus 142 can be circumferential in nature, or can span only a portion or multiple portions of the circumference of the tubular member 112.

Positioning the sensing apparatus 142 in the tubular member 112 instead of the dilator 114 may be advantageous because such positioning avoids having the sensing apparatus 142 located distal to the intraluminal medical device 116 at any point during a deployment procedure. This may be particularly advantageous in situations in which continuous monitoring from a particular location relative to the intraluminal medical device 116 is desired, or where confirmation of function from a proximal location to the intraluminal medical device 116 is desired immediately following deployment and/or concurrently with deployment of the intraluminal medical device 116. This arrangement is considered particularly advantageous for use with valve medical devices.

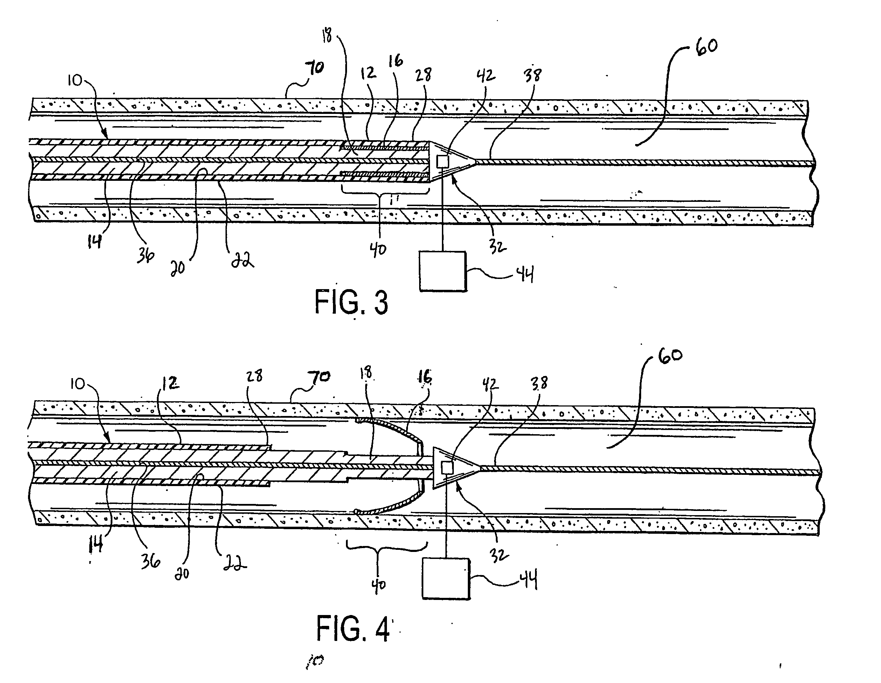

Figures 3 through 6 illustrate the delivery system 10 disposed within the lumen 60 of a body vessel 70. Each of these figures illustrates the delivery system 10 at a different stage of deployment of the intraluminal medical device 16. Figue 3 illustrates the delivery system 10 within the body vessel 70 prior to deployment. At this stage, the intraluminal medical device 16 is in its radially compressed configuration and the tubular member 12 has not yet been retracted from its position about the intraluminal medical device 16. Figure 4 illustrates the delivery system in a first stage of deployment of the intraluminal medical device. In this stage, the tubular member 12 has been retracted from its position about the intraluminal medical device 16 to a point proximal of the intraluminal medical device 16. As a result, the constraining force that maintains the intraluminal medical device 16 in its unexpanded configuration has been removed and the intraluminal medical device 16 has expanded into contact with the interior wall 72 of the body vessel 70. The dilator 14 and wireguide have not been moved from their respective positions in Figure 3. Accordingly, the distal tip 32 of the dilator 14 is disposed at a point distal to the intraluminal medical device 16.

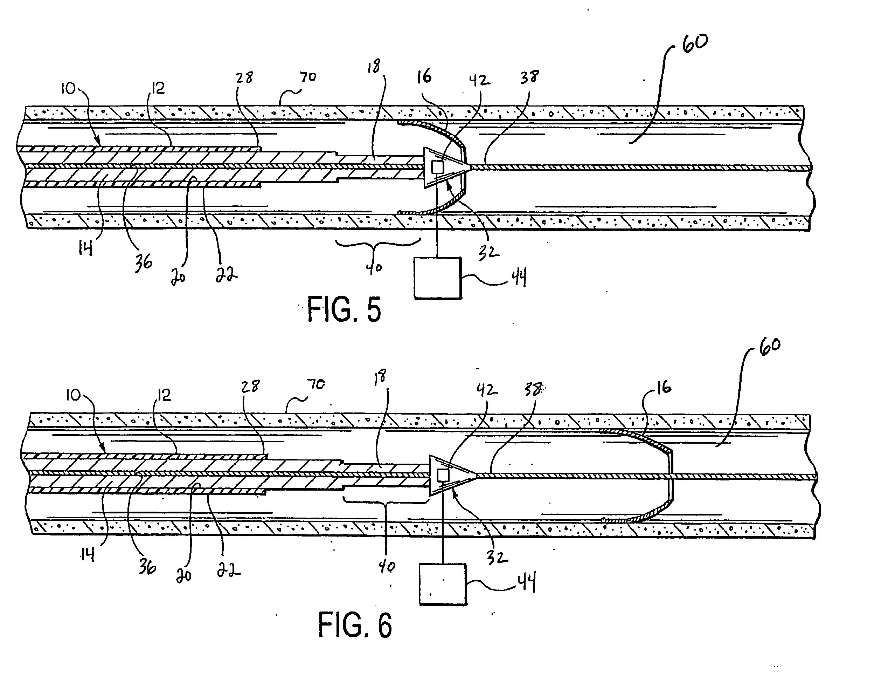

Figure 5 illustrates the delivery system 10 in a second stage of deployment of the intraluminal medical device 16. In this stage, the dilator 14 has been retracted somewhat, which is necessary for the ultimate withdrawal of the delivery system from the body vessel 70. In this stage, the distal tip 32 of the dilator 14 is disposed within a lumen of the intraluminal medical device 16.

Figure 6 illustrates the delivery system 10 in a third stage of deployment of the intraluminal medical device 16. In this stage, the dilator 14 has been retracted further. In this stage, the distal tip 32 of the dilator 14 is disposed proximal to the intraluminal medical device 16 within the vessel 70. At this point, deployment of the intraluminal medical device 16 is complete. Complete withdrawal of the delivery system 10, including the wireguide 38 can occur.

As illustrated in Figures 3 through 6, the sensing apparatus 42 communicates with a signal-receiving apparatus 44 and transmits information regarding the vessel and/or fluid parameters determined by the sensing apparatus 42 to the signal-receiving apparatus 44. The sensing apparatus 42 is advantageously in data communication with the signal-receiving apparatus at least during the illustrated stages of deployment of the intraluminal medical device 16. It is understood, however, that shorter and longer communication intervals are contemplated as being included in the invention.

The signal-receiving apparatus, which can be one or more components, is adapted to convey the information to a user in a meaningful manner. Thus, the signal-receiving apparatus 44 may include a graphical display, a digital display, an analog display, a video display, an image display, a printer, and other components adapted to convey information to a user in a meaningful manner.

A wired or wireless interface can be used between the sensing apparatus 42 and the signal-receiving apparatus 44 as desired. For example, leads can be extended from the sensing apparatus 42 through the delivery system 10 and, ultimately to the signal-receiving apparatus 44. Alternatively, a wireless interface between the sensing apparatus 42 and the signal-receiving apparatus 44 can be used, including transmission by radio waves. Also, power can be supplied to the sensing apparatus 42 via wire leads or by a battery source stored within the delivery system 10. If power is supplied to the sensing apparatus 34 by wire, wire leads can be disposed in and directed through an additional lumen (not shown) formed in the dilator 14 and running the entire length thereof to the proximal end 34.

A user can utilize the information conveyed by the signal-receiving apparatus in a variety of manners. For example, a user can utilize the information to determine and/or verify a size parameter, such as the inner diameter, of the body vessel prior to deployment of the intraluminal medical device, to confirm deployment of an intraluminal medical device, to collect information regarding a deployment of an intraluminal medical device, such as the position at which the intraluminal medical device has been deployed, to verify function of the implanted intraluminal medical device during and/or following deployment, and/or to determine whether additional steps are needed to achieve the desired result. For example, based on information regarding positioning of an intraluminal medical device, a user may decide to reposition that intraluminal medical device at the point of treatment within the body vessel or even to deploy an additional intraluminal medical device.

Delivery systems according to the invention can be used in a variety of procedures, including in the implantation of a variety of intraluminal medical devices. The sensing apparatus 42 and signal-receiving apparatus make the delivery system 10 particularly well-suited for use in procedures in which it is desirable to assess one or more vessel and/or fluid parameters prior to, during, and/or following deployment of an intraluminal medical device at a point of treatment within a body vessel.

In one exemplary use of the delivery system 10, the wireguide 38 is initially placed in the body vessel 70 of the patient by navigating a distal end of the wireguide 38 to a point just beyond the desired point of treatment. A proximal end of the wireguide 38 is left outside the body of the patient. When it is desired to insert the delivery system 10 in the body vessel 70, the proximal end of the wireguide 38 is inserted into the lumen 36 of the dilator 14. The distal end 18 of the dilator 14 is advanced along the wireguide 38, into the body vessel 70 and to the desired area of treatment.

Valve medical devices are an exemplary type of intraluminal medical device that can be implanted using a delivery system according to the invention. A valve device provides a valve for regulating the flow of fluid through a body vessel. Exemplary types of valve devices include venous valve devices, which are implanted to regulate the flow of fluid through a vessel in the vasculature, and heart valve devices, which are implanted to regulate the flow of fluid through a vessel of the heart. Following implantation of a valve device, it is desirable to confirm that the valve is providing the desired valving function, i.e., regulation of fluid flow through the body vessel in which the valve is implanted. Confirmation of function can be conducted following implantation as a separate step using an ancillary device, such as an ultrasound device. Using a delivery system according to the invention, though, the need for a separate step and/or an ancillary device to confirm valve function is eliminated. For example, a delivery system according to the invention, which includes an appropriate sensing apparatus, can be used to detect changes in fluid pressure at a point of treatment following deployment of a prosthetic valve. Regular changes in fluid pressure, and the achievement of particular values, may indicate proper functioning of the implanted valve device. In this embodiment, the sensing apparatus 42 can detect fluid pressure and changes in fluid pressure and communicate information relating to the fluid pressure determinations to the signal-receiving apparatus, allowing the user to confirm valve function.

Other parameters can also be used to confirm valve function. For example, visualization of the point of treatment, as described above, can verify valve function by providing the user with specific visual indications of valving action.

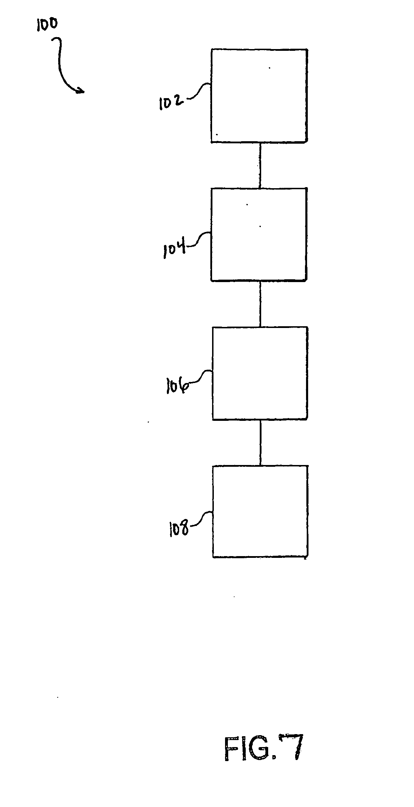

Figure 7 illustrates an exemplary method 100 of implanting an intraluminal medical device according to the invention. The order of steps illustrated and described herein is exemplary in nature and, as a result, is not considered necessary or critical. In one step 102, a delivery system including an intraluminal medical device is provided. In another step 104, a distal end of the.delivery system is inserted in a body vessel of a patient. In another step 106, one or more vessel and/or fluid parameters is determined. In another step 108, the intraluminal medical device is deployed from the delivery system at a point of treatment in the body vessel. The delivery system can then be removed from the body vessel of the patient.

In the method of implanting an intraluminal medical device, the step 106 of determining one or more vessel and/or fluid parameters can be conducted prior to, during, and/or following the step 108 of deploying the intraluminal medical device.

In exemplary methods, an initial sizing step can be conducted prior to the step 104 in which the delivery system is inserted into the body vessel. In these methods, an appropriate vessel sizing device and/or technique, such as venography, is conducted priorto insertion of the delivery device. This pre-sizing step provides initial sizing information that can be confirmed with the sensing apparatus of the delivery system according to the invention during the subsequent step 106 of determining a vessel parameter, or during a separate confirmation step that involves comparing the vessel parameter information to the initial sizing information. This method is particularly advantageous in procedures in which convention sizing techniques have limitations that may lead to sizing errors that are determined upon subsequent implantation of an intraluminal medical device. For example, conventional venography techniques are somewhat limited for sizing body vessels because they produce a two dimensional venogram that may or may not provide allow for accurate determination of vessel size. Confirmation of vessel size at the intended point of treatment from within the body vessel can reduce the possibility of improperly sized intraluminal medical devices. Confirmation of vessel size using this method can also reduce and/or eliminate the use of additional materials, such as intraluminal medical devices and entire delivery systems, which can be necessitated by sizing errors.

In exemplary embodiments, the intraluminal medical device comprises a valve device, such as a venous valve device or a heart valve device.

The foregoing detailed description provides exemplary embodiments of the invention and includes the best mode for practicing the invention. These embodiments are intended only to serve as examples of the invention; and not to limit the scope of the invention, or its protection, in any manner.

## Claims

1. A delivery system (10,110) for implanting an intraluminal medical device (16,116) within a body vessel, said delivery system comprising:
an elongate tubular member (12,112) having a first distal end (28) adapted for insertion into a body vessel;
a dilator (14,114) disposed in the tubular member (12,112) and having a second distal end (18,118) adapted for insertion into a body vessel (70) the dilator (14,114) cooperating with the tubular member (12,112) to define a device chamber (40) between the tubular member (12,112) and the dilator;
**characterised in that** the delivery system comprises an expandable valve medical device disposed in the device chamber (40); and
a sensing apparatus (42,142) disposed in the second distal end (18,118) of the dilator (14,114) or in or on the dilator (14,114) adjacent to the device chamber (40), the sensing apparatus adapted to determine at least one of a vessel parameter and a fluid parameter and to transmit information relating to the at least one of a vessel parameter and a fluid parameter to a signal-receiving apparatus.

2. The delivery system according to Claim 1, wherein the sensing apparatus (42,142) comprises an imaging apparatus.

3. The delivery system according to Claim 1, wherein the sensing apparatus (42,142) comprises one of a fluid pressure sensor, a biochemical sensor, and a temperature sensor.

4. The delivery system according to Claims 1,2 or 3 wherein the sensing apparatus (42,142) is disposed distal to the device chamber (40).

5. The delivery system according to any preceding claim and wherein the expandable valve device comprises a venous valve medical device.

6. The delivery system according to claims 1,2,3 or 4 wherein the expandable valve device comprises a heart valve medical device.

## Patentansprüche

1. Abgabesystem (10, 110) zur Implantation eines intraluminalen Medizinprodukts (16, 116) in einem Körpergefäß, wobei das Abgabesystem Folgendes umfasst:
ein längliches röhrenförmiges Element (12, 112) mit einem ersten distalen Ende (28), das in einen Körper eingeführt werden kann; einen Dilatator (14, 114), der im röhrenförmigen Element (12, 112) angeordnet ist und
ein zweites distales Ende (18, 118) aufweist, das in ein Körpergefäß (70) eingeführt werden kann, wobei der Dilatator (14, 114) mit dem röhrenförmigen Element (12, 112) zusammenwirkt, um eine Produktkammer (40) zwischen dem röhrenförmigen Element (12, 112) und dem Dilatator zu definieren, **dadurch gekennzeichnet, dass** das Abgabesystem eine expandierbare medizinische Klappenvorrichtung umfasst, die in der Produktkammer (40) angeordnet ist, und dass ein Messgerät (42, 142) im zweiten distalen Ende (18, 118) des Dilatators (14, 114) oder im oder auf dem Dilatator (14, 114) neben der Produktkammer (40) angeordnet ist, wobei das Messgerät zumindest einen Gefäßparameter und/oder zumindest einen Flüssigkeitsparameter bestimmen kann, und
Informationen, die sich auf den zumindest einen Gefäßparameter und/oder zumindest einen Flüssigkeitsparameter beziehen, an ein Signalempfangsgerät übertragen kann.

2. Abgabesystem nach Anspruch 1, worin das Messgerät (42, 142) ein Bildgebungsgerät umfasst.

3. Abgabesystem nach Anspruch 1, worin das Messgerät (42, 142) einen Flüssigkeitsdrucksensor, einen biochemischen Sensor oder einen Temperatursensor umfasst.

4. Abgabesystem nach Anspruch 1, 2 oder 3, worin das Messgerät (42, 142) distal von der Produktkammer (40) angeordnet ist.

5. Abgabesystem nach einem der vorhergehenden Ansprüche, worin die expandierbare Klappenvorrichtung eine medizinische Venenklappenvorrichtung umfasst.

6. Abgabesystem nach Anspruch 1, 2, 3 oder 4, worin die expandierbare Klappenvorrichtung eine medizinische Herzklappenvorrichtung umfasst.

## Revendications

1. Système de pose (10,110) pour implanter un dispositif médical intraluminal (16,116) à l'intérieur d'un vaisseau corporel, ledit système de pose comportant :
un organe tubulaire allongé (12,112) présentant une première extrémité distale (28) conçue pour être introduite dans un vaisseau corporel ;
un dilatateur (14,114) disposé dans l'organe tubulaire (12,112) et présentant une deuxième extrémité distale (18,118) conçue pour être introduite dans un vaisseau corporel (70), le dilatateur (14,114) coopérant avec l'organe tubulaire (12,112) pour définir une chambre de dispositif (40) entre l'organe tubulaire (12,112) et le dilatateur ;
**caractérisé en ce que** le système de pose comporte un dispositif médical expansible disposé dans la chambre de dispositif (40) ; et
un appareil de détection (42,142) disposé dans la deuxième extrémité distale (18,118) du dilatateur (14,114) ou dans ou sur le dilatateur (14,114) à côté de la chambre de dispositif (40), l'appareil de détection étant adapté de façon à déterminer au moins un paramètre de vaisseau et un paramètre de fluide et à transmettre les informations relatives audit au moins un paramètre de vaisseau et ou paramètre de fluide à un appareil recevant un signal.

2. Système de pose selon la revendication 1, dans lequel l'appareil de détection (42,142) comporte un appareil d'imagerie.

3. Système de pose selon la revendication 1, dans lequel l'appareil de détection (42,142) comporte un détecteur de pression de fluide, un détecteur biochimique ou un détecteur de température.

4. Système de pose selon la revendication 1, 2 ou 3 dans lequel l'appareil de détection (42,142) est disposé distalement par rapport à la chambre de dispositif (40).

5. Système de pose selon l'une quelconque des revendications précédentes et dans lequel le dispositif à valve expansible comporte un dispositif médical à valve veineuse.

6. Système de pose selon la revendication 1, 2, 3 ou 4 dans lequel le dispositif à valve expansible comporte un dispositif médical à valve cardiaque.
